# EUROPEAN PATENT APPLICATION

(11) **EP 4 729 971 A1**
(43) Date of publication of application: **22.04.2026**
(21) Application number: 24839907.3
(22) Date of filing: 28.05.2024
(51) Int. Cl.: G01R 33/00, G01R 23/12, G01R 33/035, A61B 5/245, G01C 21/08

(54) **QUANTUM SENSOR SYSTEM**

(30) Priority: 13.07.2023 KR 20230091385
(71) Applicant: LG Electronics Inc., Yeongdeungpo-gu Seoul 07336 (KR)
(72) Inventor: JEON, Dongjae, Seoul 06772 (KR); KIM, Seonghyok, Seoul 06772 (KR); LEE, Keunyoung, Seoul 06772 (KR); LEE, Sungdan, Seoul 06772 (KR); KIM, Hongyeol, Seoul 06772 (KR); LEE, Myeongwon, Seoul 06772 (KR)
(74) Representative: Vossius & Partner Patentanwälte Rechtsanwälte mbB
(86) International application number: PCT/KR2024/007227
(87) International publication number: WO 2025/014090

(57) **Abstract**

This quantum sensor system comprises: a magnetic sensor module disposed spaced apart from a magnetic device to measure a magnetic field from the magnetic device and having a second temperature sensor; and a main circuit operably coupled to the magnetic sensor module and having a first temperature sensor. The main circuit is configured to measure the magnetic field in a specific frequency band on the basis of the temperature value difference between a first temperature value measured by the first temperature sensor and a second temperature value measured by the second temperature sensor.

## Description

### Technical Field

The present disclosure relates to a quantum sensor system. More specifically, the present disclosure relates to a quantum sensor system for measuring a magnetic field.

### Background Art

A diamond crystal composed of carbon atoms has a lattice defect when the carbon atoms are replaced with other types of atoms. The diamond crystal having the lattice defect becomes a nitrogen-vacancy center, in which one carbon atom is replaced with a nitrogen atom, and the adjacent carbon atom site is removed, leaving an unoccupied space.

A nitrogen-vacancy (NV) diamond structure possesses an electron spin with a spin number S = 1, thereby allowing three spin states m_s of +1, 0, and -1. In a case where an external magnetic field is not present in the axial direction of the nitrogen-vacancy center within a diamond, spin quanta in spin states m_s of +1 and -1 become superposed, thereby existing at substantially the same energy level.

When light having a wavelength of 532 nm is directed onto the NV diamond structure, a quantum system in a spin state m_s = 0 is excited and then returns to the ground state while emitting light having a wavelength of 600 nm or greater. Quantum systems in the spin states m_s = +1 and m_s = -1 are excited and then return to the ground state while transitioning to the spin state m_s = 0.

When an external magnetic field exists in the axial direction of the nitrogen-vacancy center within the NV diamond structure, the superposition of the spin quantum states m_s = +1 and m_s = -1 of the nitrogen-vacancy center disappears due to the Zeeman effect, and the spin quantum states exist at different energy levels. Accordingly, a spin quantum system in the nitrogen-vacancy center has two resonance frequencies corresponding to spin transitions between the spin state m_s = 0 and a spin state m_s = +1, or between the spin state m_s = 0 and the spin state m_s = -1. A difference between two resonance frequencies is proportional to the magnitude of the external magnetic field.

A quantum sensor system having the NV diamond structure may experience an error in magnetic-field measurement due to a change in temperature in the vicinity of the NV diamond structure. Specifically, there is a disadvantage in that light having a specific wavelength is directed onto the NV diamond structure, the temperature in the vicinity of the NV diamond structure is increased, thereby causing an error in the magnetic-field measurement.

### Disclosure of Invention

### Technical Problem

One object of the present disclosure is to provide a quantum sensor system for measuring a magnetic field through an NV diamond structure.

Another object of the present disclosure is to address a problem in which an error occurs in measuring a magnetic field due to a change in temperature in the vicinity of an NV diamond structure.

A further object of the present disclosure is to address a problem in which an error occurs in measuring a magnetic field due to an increase in temperature in the vicinity of an NV diamond structure when light having a specific wavelength is directed onto an NV diamond structure.

### Solution to Problem

According to one aspect of the present disclosure, there is provided a quantum sensor system including: a magnetic sensor module arranged to be spaced apart from a magnetic device in such a manner as to measure a magnetic field from the magnetic device and including a second temperature sensor; and a main circuit operatively coupled to the magnetic sensor module and including a first temperature sensor. In the quantum sensor system, the main circuit is configured to measure the magnetic field in a specific frequency band on the basis of a temperature value difference between a first temperature value measured by the first temperature sensor and a second temperature value measured by the second temperature sensor.

According to an embodiment, in the quantum sensor system, the magnetic sensor module may include: a first substrate on which a light source is arranged; and a second substrate which is arranged to be spaced apart from the first substrate in a direction along a perpendicular axis and on which an NV diamond structure and the first temperature sensor are arranged. In the quantum sensor system, a photodetector arranged to be spaced apart from the second substrate in the direction along the perpendicular axis and configured to detect a signal emitted from the NV diamond structure may be provided on the magnetic sensor module or the main circuit.

According to an embodiment, the quantum sensor system may further include a lock-in amplifier configured to amplify a signal from the photodetector and output the resulting output signal. In the quantum sensor system, when the temperature value difference is less than a threshold temperature difference, the main circuit may be configured to measure the magnetic field that varies with a slope of the output signal that varies with a change in frequency in a first band.

According to an embodiment, in the quantum sensor system, when the temperature value difference is equal to or greater than the threshold temperature difference, the main circuit may measure the magnetic field according to the slope of the output signal that varies with the change in frequency in a second frequency band. In the quantum sensor system, the second frequency band may be a frequency band narrower than the first frequency band.

According to an embodiment, in the quantum sensor system, the main circuit may measure the magnetic field in the specific frequency band or may change the specific frequency band for measuring the magnetic field on the basis of an updated temperature value difference between the updated first temperature value and the second temperature value.

According to an embodiment, in the quantum sensor system, when the updated temperature value difference is less than the threshold temperature value, the main circuit may control the magnetic sensor module in such a manner as to measure the magnetic field at a resonant frequency of the magnetic sensor module.

According to an embodiment, in the quantum sensor system, when the updated temperature value difference is equal to or greater than the threshold temperature value, the main circuit may control the magnetic sensor module in such a manner that a resonant frequency of the magnetic sensor module is shifted according to the updated temperature value difference, and may control the magnetic sensor module in such a manner as to measure the magnetic field at the shifted resonant frequency.

According to an embodiment, in the quantum sensor system, the magnetic sensor module may further include a resonator formed on the first substrate on which the light source is arranged, and configured to radiate an RF signal. According to an embodiment, in the quantum sensor system, the magnetic sensor module may further include an RF generator generating the RF signal and applying the generated RF signal to the resonator. In the quantum sensor system, the main circuit may control the RF generator in such a manner as to generate the RF signal at the shifted resonant frequency.

According to an embodiment, in the quantum sensor system, the main circuit may compute a frequency shift value according to the updated temperature value difference and may compute a value by dividing the computed frequency shift value by a minimum frequency sweep interval. In the quantum sensor system, the main circuit may control the RF generator in such a manner as to generate the RF signal at the shifted resonant frequency corresponding to an integer multiple of frequency-step spacings that is closest to the computed value, and may control an operational frequency band of the lock-in amplifier in such a manner as to measure the magnetic field at the shifted resonant frequency.

According to an embodiment, in the quantum sensor system, when the updated temperature value difference is equal to or greater than the threshold temperature value, the magnetic sensor module may be configured to control the RF generator on the basis of the updated temperature value difference in such a manner as to change a first resonant frequency of the resonator arranged on the first substrate.

According to an embodiment, in the quantum sensor system, the first temperature sensor may be arranged within a processor of the main circuit or within a measurement module that measures temperature. In the quantum sensor system, the second temperature sensor may be arranged within the processor of the main circuit or within a second measurement module that measures temperature. In the quantum sensor system, the second temperature sensor may be arranged on one side or the other side of the NV diamond structure.

According to an embodiment, in the quantum sensor system, the NV diamond structure may be arranged in a slot region of the second substrate in such a manner that light emitted from the light source through the slot region of the second substrate is incident thereon.

According to an embodiment, in the quantum sensor system, the light source may be an LED light source operating in a visible light region, a laser light source, an infrared light source, or an ultraviolet light source.

According to an embodiment, in the quantum sensor system, the first temperature sensor may detect a change in outdoor air temperature due to heat generated from a processor of the main circuit. In the quantum sensor system, the main circuit may control the magnetic sensor module in such a manner that a resonant-frequency shift value of the NV diamond structure caused by the change in outdoor air temperature is decreased. In the quantum sensor system, as the resonant-frequency shift value is decreased, the sensitivity of the measurement of the magnetic field from the magnetic device may be decreased.

According to an embodiment, in the quantum sensor system, each of the first temperature sensor and the second temperature sensor may be configured as at least one of the following: a silicon temperature sensor utilizing the dependence of a base-emitter voltage V_BE of a transistor on temperature, a thermistor, an RTD, or a thermocouple.

According to an embodiment, in the quantum sensor system, the main circuit may track a shift in the resonant frequency due to a single quantum spin structure associated with a single photon in the NV diamond structure in conjunction with the first and second temperature sensors, and may detect a maximum signal magnitude of the resonator during oscillation caused by the single photon according to the shift in the resonant frequency. In the quantum sensor system, the main circuit may minimize control errors associated with the single photon by adjusting the resonant frequency on the basis of the maximum signal magnitude and the temperature value difference.

According to an embodiment, in the quantum sensor system, the main circuit may track a shift in the resonant frequency caused by a multiplicity of quantum spin structures associated with multiple photons of an ensemble NV diamond structure within the NV diamond structure in conjunction with the first and second temperature sensors, and may detect a maximum signal magnitude of the resonator during oscillation caused by the multiple photons according to the shift in the resonant frequency. In the quantum sensor system, the main circuit may minimize control errors associated with the multiple photons by adjusting the resonant frequency on the basis of the maximum signal magnitude and the temperature value difference.

According to an embodiment, in the quantum sensor system, the main circuit may execute spin control of the diamond structure at the resonant frequency when an amount of change in temperature measured by the second temperature sensor, which is arranged on the second substrate in a manner that is adjacent to the NV diamond structure, is less than a threshold value. In the quantum sensor system, the main circuit may execute the spin control of the diamond structure at a frequency that results from adding the amount of change in frequency, which is in proportion to the amount of change in temperature, to the resonant frequency, when the amount of change in temperature measured by the second temperature sensor is equal to or greater than the threshold value.

According to an embodiment, the quantum sensor system may further an electromagnetic wave blocking sheet arranged on a substrate of the main circuit in such a manner to shield power noise. In the quantum sensor system, the electromagnetic wave blocking sheet may be arranged adjacent to the periphery of the photodetector. In the quantum sensor system, the electromagnetic wave blocking sheet may be a ferrite sheet or a sheet having different physical properties than the ferrite sheet.

According to an embodiment, in the quantum sensor system, the main circuit may detect a rate of decrease over time in an amount of light of an optical signal from the light source through a residual fitting algorithm, in conjunction with the photodetector. In the quantum sensor system, the main circuit may control initial driving of the light source and a situation in which the amount of light is attenuated, through a residual analysis algorithm, in such a manner as to compensate for the decrease over time in the amount of light of the optical signal.

### Advantageous Effects of Invention

According to the present disclosure, errors in measurement of a magnetic field due to a change in ambient temperature can be decreased through a quantum sensor system for measuring the magnetic field through an NV diamond structure.

According to the present disclosure, the errors in the measurement of the magnetic field due to the change in ambient temperature can be decreased through a first temperature sensor provided on a main circuit and a second temperature sensor arranged adjacent to the NV diamond structure.

According to the present disclosure, the errors in the measurement of the magnetic field due to an increase in temperature in the vicinity of the NV diamond structure, which is caused by directing light having a specific wavelength, can be decreased through the first temperature sensor provided on the main circuit and the second temperature sensor arranged adjacent to the NV diamond structure.

### Brief Description of Drawings

FIG. 1 is a set of views, two of which illustrate an NV diamond structure used in a quantum sensor system and one of which illustrates a result of electron spin analysis.
FIG. 2 is a view illustrating an energy band diagram of the NV diamond structure and emission intensity resulting from a spin state.
FIG. 3 is a set of views, one of which illustrates a change in an energy diagram and the other of which illustrates a fluorescence change on a per-frequency basis, the changes resulting from application of a magnetic field.
FIGS. 4 and 5 are graphs illustrating a resonance frequency of the NV diamond structure that varies with temperature in the quantum sensor system according to the present disclosure.
FIG. 6 is a diagram illustrating the configuration of the quantum sensor system according to the present disclosure.
FIG. 7 is a diagram illustrating a magnetic sensor module formed to have a shielding structure for shielding power switching noise, according to the present specification.
FIG. 8 is a diagram illustrating the configuration of the quantum system including a multiplicity of components, according to a first embodiment. FIG. 9 is a diagram illustrating the configuration of the quantum system including a multiplicity of components, according to a second embodiment.
FIG. 9 is a diagram illustrating the configuration of the quantum system including a multiplicity of components, according to a second embodiment.
FIG. 10 is a graph illustrating an output voltage that varies with a change in frequency of the NV diamond structure.
FIG. 11 is a table illustrating output voltages that result from frequency shifting under first and second temperature conditions on Curved Line 1 (Gr1) and Curved Line 2 (Gr2).
FIG. 12 is a flowchart illustrating a method of controlling the quantum sensor system for measuring the magnetic field in different ways on the basis of an updated temperature difference between a first temperature value and a second temperature value.
FIG. 13 is a graph illustrating light intensity that varies with a spin state in the quantum sensor system according to the present disclosure.
FIG. 14 is a set of conceptual diagrams, each illustrating a method of measuring continuous magnetic fields using an interferometer according to a first embodiment.
FIG. 15 is a set of conceptual diagrams, each illustrating a method of measuring an oscillation period according to a second embodiment.
FIG. 16 is a set of graphs, one of which illustrates a decrease over time in an amount of light of an optical signal in the quantum sensor system according to the present disclosure, and the other of which illustrates compensation for the decrease over time in the amount of light.

### Mode for the invention

The technology disclosed in the present specification applies to a quantum sensor system measuring a magnetic field. However, the technology disclosed in the present disclosure is not limited thereto and may also apply to a quantum sensor system measuring all magnetic fields in which the technical concept of the technology can find application.

It is noted that technical terms used in the present specification are only for describing specific embodiments and are not intended to impose any limitation on the scope of the present disclosure. In addition, unless otherwise specifically defined, the technical terms used in the present specification should be construed as having the meanings generally understood by those of ordinary skill in the art to which the present disclosure pertains. These terms should not be construed either too broadly or too narrowly. In addition, when the technical term used in the present specification does not precisely reflect the technical concept of the present disclosure, it should be construed to have the meaning generally accepted by those of ordinary skill in the art. In addition, general terms used in the present specification should be interpreted based on their standard dictionary meanings or as understood within the context of this disclosure, without applying an unduly narrow construction.

In addition, the term used in the present specification, although expressed in the singular, is construed to have its plural form as well unless the context clearly indicates otherwise. Throughout the present specification, the terms such as 'is configured to include' or 'includes' should not be construed as requiring all constituent elements or all steps described herein. Rather, these terms should be construed to allow for the omission of one or more elements or steps, as well as the inclusion of one or more additional elements or steps.

In addition, for descriptive convenience, the terms 'module' and 'unit' are interchangeably used in the present specification to refer to constituent elements, without implying any difference in meaning or interpretation.

In addition, the terms that indicate ordinal numbers in the present specification, such as 'first,' 'second,' and so forth, are used to describe various constituent elements for purposes of distinction without imposing any limitation on them. These terms are used merely to distinguish among constituent elements having similar functions. For example, a first constituent element may be referred to as a second constituent element without departing from the scope of claims. Similarly, the second constituent element may be referred to as the first constituent element.

Preferred embodiments according to the present disclosure are described in detail below with reference to the accompanying drawings. For clarity, like constituent elements are designated the same reference numerals, and repetitive descriptions thereof are omitted.

In addition, if it is determined that a detailed description of technology known in the related art would hinder a clear understanding of the nature and gist of the present disclosure, such description is omitted. In addition, the accompanying drawings are provided solely to facilitate understanding of the technical idea disclosed in the present specification. It should be noted that the accompanying drawings are not construed as imposing any limitation on the underlying technical idea.

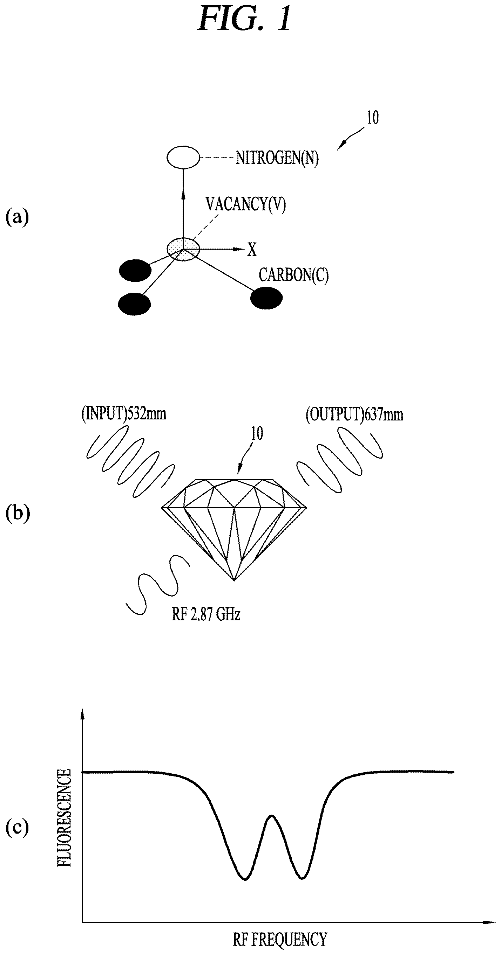

In this regard, FIG. 1 is a set of views, two of which illustrate an NV diamond structure used in the quantum sensor system and one of which illustrates a result of electron spin analysis. (a) of FIG. 1 illustrates the NV diamond structure. (b) of FIG. 1 illustrates the respective wavelengths of an input signal and an output signal that are incident on the NV diamond structure. (c) of FIG. 1 illustrates a change in the electron spin of the NV diamond structure due to the characteristics of a surrounding magnetic field or to a change in temperature.

With reference to (a) FIG. 1, an NV diamond structure 10 may be configured to contain a nitrogen (N) atom (N) and a vacancy (V). Quantum qubits may be controlled using a defect in the atomic structure in the diamond associated with the NV diamond structure 10. The NV diamond structure 10 is formed to include an NV center in which one nitrogen atom is arranged among the densely packed carbon atoms constituting the diamond, and in which a vacancy (V) is formed. In the NV diamond structure 10, the electron spins may be used as qubits. The NV diamond structure 10 may be used to enable quantum computers, quantum communication, and quantum sensors.

Other quantum computer technologies, such as superconductor systems or ion-trap methods, must be used at ultra-low temperatures or within an ultra-high-vacuum environment to manipulate sensitive qubits in a stable environment. In contrast, the diamond NV center method may be used even in room-temperature conditions due to the surrounding solid-state carbon lattice. The diamond NV center method may be implemented without employing a large-scale cooling apparatus required for a superconducting quantum computer system.

With reference to (b) of FIG. 1, an RF signal at a first frequency (e.g., 2.87 GHz) may be input into the NV diamond structure 10. An optical signal having a first wavelength within a predetermined range centered at 532 nm may be input into (applied to) the NV diamond structure 10. A second optical signal having a second wavelength longer than the first wavelength may be output from the NV diamond structure 10. In this regard, the second optical signal having the second wavelength within a predetermined range centered at 637 nm may be output (emitted) from the NV diamond structure 10. Accordingly, the quantum sensor system may be enabled by employing an optical system configuration for detecting or analyzing an RF signal for generating or controlling a quantum state.

With reference to (c) of FIG. 1, a change in the electron spin of the NV center of the NV diamond structure occurs due to the characteristics of the surrounding magnetic field. The characteristics of the surrounding magnetic field cause the phenomenon in which resonance frequencies are separated due to Zeeman splitting. Therefore, the characteristics of the magnetic field surrounding the NV diamond structure may be measured by detecting a change in the resonance frequencies.

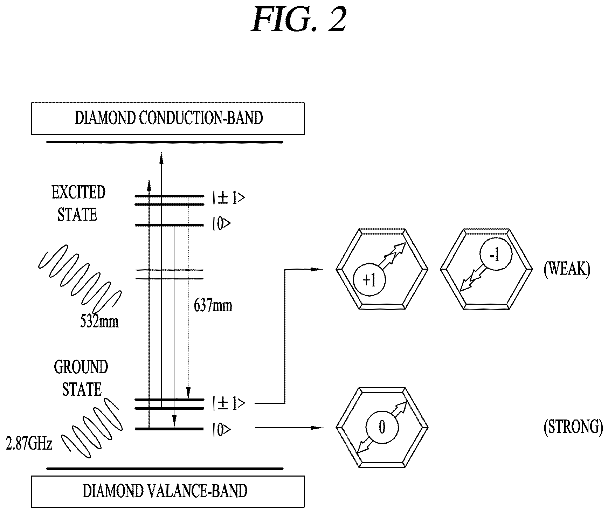

FIG. 2 is a view illustrating an energy band diagram of the NV diamond structure and emission intensity resulting from a spin state. With reference to FIGS. 1 and 2, when the optical signal having the first wavelength (e.g., 532 nm) is applied to the NV diamond structure 10 in a ground state, the NV diamond structure 10 may transition to an excited state. Accordingly, the NV diamond structure 10 may undergo an energy band transition from a valence band to a conduction band.

The NV diamond structure 10 in the excited state may output the optical signal having the second wavelength (e.g., 637 nm), thereby transitioning to the ground state. Accordingly, the NV diamond structure 10 may undergo the energy band transition from the conduction band to the valence band. Energy levels associated with the NV center of the NV diamond structure 10 may be arranged within a bandgap between the conduction band and the valence band.

At this point, when the quantum state of the NV diamond structure 10, which indicates a transition from the excited state to the ground state, is ±1, the emission intensity of the NV diamond structure 10 may be determined to be lower at the second wavelength than a threshold value. In this regard, the emission intensity of the NV diamond structure 10 may be observed to have a higher value in a band of wavelengths longer than the second wavelength. At this point, when the quantum state of the NV diamond structure 10, which indicates a transition from the excited state to the ground state, is 0, the emission intensity of the NV diamond structure 10 may be observed to have a higher value at the second wavelength than the threshold value.

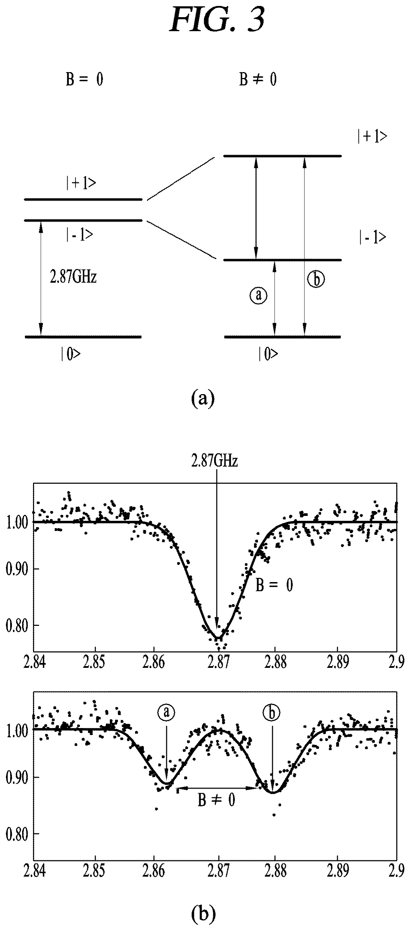

FIG. 3 is a set of views, one of which illustrates a change in an energy diagram and the other of which illustrates a fluorescence change on a per-frequency basis, the changes resulting from application of a magnetic field. (a) of FIG. 3 illustrates a change in an energy diagram that results from the application of a magnetic field. (b) of FIG. 3 illustrates a fluorescence change on a per-frequency basis that results from the application of a magnetic field. With reference to (a) FIG. 3, when a magnetic field B is applied, a gap between energy band structures increases due to the Zeeman splitting caused by the magnetic field B. With reference to (b) of FIG. 3, when the magnetic field is zero, a single resonant frequency of 2.87 GHz is observed. In contrast, when the magnetic field B is applied, dual resonances occur at frequencies lower and higher than 2.87 GHz due to the applied magnetic field B. Therefore, through the analysis of the change in the resonant frequency, measurement of the surrounding magnetic field becomes possible.

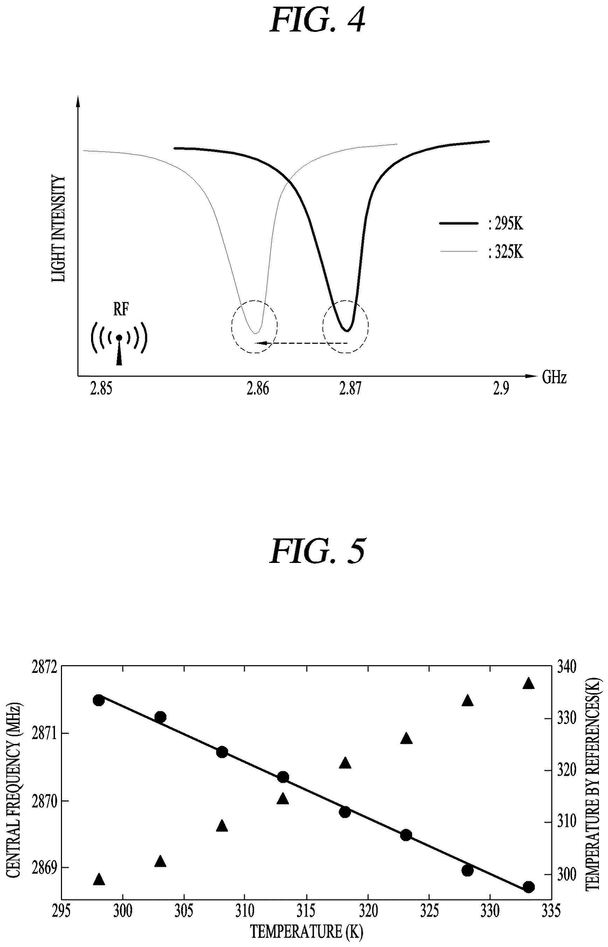

The quantum sensor system according to the present disclosure will be described below. FIGS. 4 and 5 are graphs illustrating a resonant frequency of the NV diamond structure that varies with temperature in the quantum sensor system according to the present disclosure.

With reference to FIGS. 1 and 4, as the temperature of the region in which the NV diamond structure 10 is arranged increases, the resonant frequency is shifted toward a lower frequency. For example, if the temperature is 295 K, the resonant frequency is observed to be 2.87 GHz. Conversely, when the temperature increases to 325 K, the resonant frequency is observed to be 2.86 GHz.

With reference to FIGS. 1, 4, and 5, as the temperature of the region in which the NV diamond structure 10 is arranged increases, the resonant frequency is shifted toward a lower frequency. Specifically, as the temperature increases, the resonant frequency is shifted toward a lower frequency at a rate of 74.2 kHz/K.

The shift of the central frequency of the NV diamond structure 10 has a linear relationship with the change in temperature. When an ultra-low temperature section is reached, the adjacent spacing between nitrogen-vacancy-carbon constituents reaches a threshold value, thereby causing a loss of linearity. However, since a primary advantage of the NV diamond structure 10 is a room-temperature quantum characteristic, the NV diamond structure 10 may be practically used in a room-temperature range.

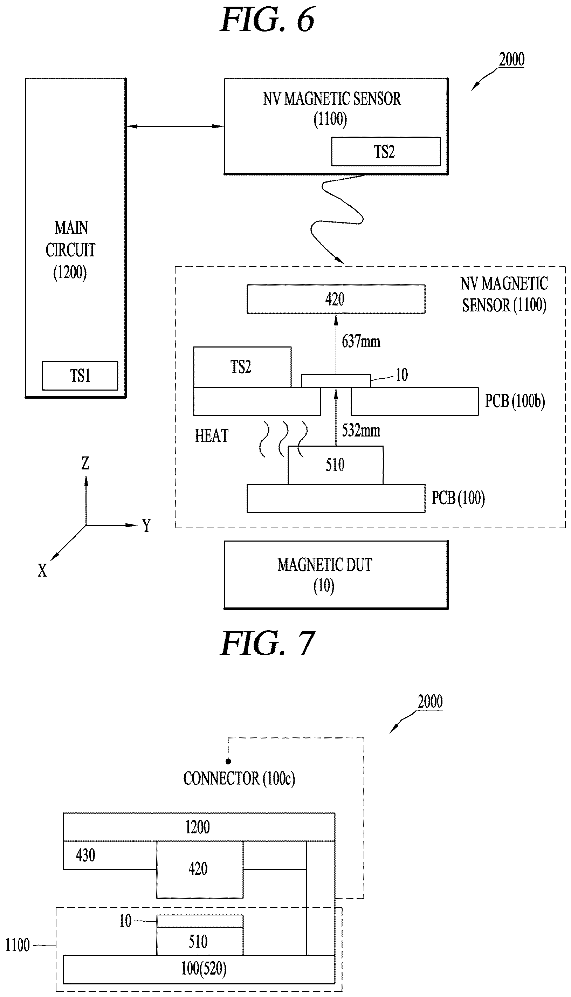

FIG. 6 is a diagram illustrating the configuration of the quantum sensor system according to the present disclosure. With reference to FIG. 6, a quantum sensor system 2000 may be configured to include a magnetic sensor module 1100 and a main circuit 1200.

The magnetic sensor module 1100 may be arranged to be spaced apart from a magnetic device 1 in such a manner as to measure a magnetic field from the magnetic device 1. The sensor module 1100 may be configured as a Printed Circuit Board (PCB). The magnetic device 1 may be a portion of the human body that emits a human-originated magnetic field, as well as an arbitrary electronic device that emits a magnetic field. The magnetic field emitted from the magnetic device 1 may be a direct-current magnetic field, but is not limited thereto. The magnetic field may also be an alternating-current magnetic field. The magnetic sensor module 1100 may include a second temperature sensor TS2 in such a manner as to measure the change in temperature.

The main circuit 1200 may be operatively coupled to the magnetic sensor module 1100. The main circuit 1200 may be configured as a Micro Control Unit (MCU) or an Application Processor (AP), but is not limited thereto. The main circuit 1200 may be modified depending on the application. The magnetic sensor module 1100 may be configured as a substrate, such as a PCB, and the main circuit 1200 may be configured as a substrate on which a processor, such as an AP, is mounted. The respective substrates of the magnetic sensor module 1100 and the main circuit 1200 may be operatively coupled to transmit and receive signals through a cable.

The main circuit 1200 may be configured to operate in conjunction with the second temperature sensor TS2 of the magnetic sensor module 1100. The main circuit 1200 may be configured to include a first temperature sensor TS1 to measure the change in temperature.

The quantum sensor system, which operates with low power, is required to minimize a distance between a light source and the NV diamond structure to reduce light loss. Accordingly, the quantum sensor system operating with low power may be more susceptible to heat generated by the light source. To address this problem, the quantum sensor system 2000 according to the present disclosure may be configured to measure a magnetic field on the basis of a temperature difference between the first temperature sensor TS1 of the main circuit 1200 and the second temperature sensor TS2 of the magnetic sensor module 1100.

In this regard, the main circuit 1200 may be configured to measure a magnetic field in a specific frequency band on the basis of a temperature difference value between a first temperature value measured by the first temperature sensor TS1 and a second temperature value measured by the second temperature sensor TS2. The main circuit 1200 may measure a magnetic field from the magnetic device 1 in a specific frequency band on the basis of the temperature difference value between the first temperature value and the second temperature value.

When a shift amount of the resonant frequency of the magnetic sensor module 1100 is at or above a threshold value, the main circuit 1200 may control a change in the resonant frequency on the basis of the threshold value. The main circuit 1200 may control a change in the resonant frequency of an RF signal from the magnetic sensor module 1100 on the basis of the shifted resonant frequency. To this end, the main circuit 1200 may control a change in the resonant frequency of a resonator of the magnetic sensor module 1100 or a change in the oscillation frequency of an oscillator thereof.

The magnetic sensor module 1100 may be configured to include a first substrate 100, a second substrate 100b, and a photodetector 420. The photodetector 420 may be configured as a photodiode, but is not limited thereto. The photodetector 420 is not limited to being arranged in the magnetic sensor module 1100. Depending on the application, the photodetector 420 may also be positioned in the main circuit 1200.

A light source 510 may be arranged on the first substrate 100. The light source 510 may be configured to emit the optical signal having the first wavelength. The light source 510 may be configured to emit an optical signal having a wavelength within a predetermined range centered at 532 nm. The light source 510 may be formed as an LED light source operating in a visible light region, a laser light source, an infrared light source, or an ultraviolet light source.

The second substrate 100b may be arranged to be spaced apart in a direction along a perpendicular axis (e.g., the Z-axis) from the first substrate 100. The second substrate 100b may be arranged under the first substrate 100 in the Z-axis direction and in parallel with the first substrate 100. The NV diamond structure 10 and the second temperature sensor TS2 may be arranged on the second substrate 100b. The second temperature sensor TS2 may be configured to detect the change in temperature caused by heat generated from the light source 510. The temperature sensor TS2, arranged on the second substrate TS2, may detect the change in temperature caused by the heat generated from the light source 510 arranged on top of the first substrate TS1.

The NV diamond structure 10 may be arranged in a slot region SR of the second substrate 100b in such a manner that light emitted from the light source 510 through the slot region SR of the second substrate 100b is incident thereon. The slot region SR of the second substrate 100b may also be formed as a dielectric region having a dielectric constant equal to or less than a predetermined value in such a manner that light from the light source 510 is incident thereon.

The photodetector 420 may be arranged to be spaced apart in the direction along the perpendicular axis (e.g., the Z-axis) from the second substrate 100b. The photodetector 420 may be arranged on an upper portion of the first substrate 100 in the Z-axis direction and in parallel with the second substrate 100b. The photodetector 420 may be configured to detect a signal emitted from the NV diamond structure 10 arranged on top of the first substrate 100. The NV diamond structure 10 may be configured to emit the second optical signal having the second wavelength longer than the first wavelength. The NV diamond structure 10 may be configured to emit the second optical signal having a wavelength within a predetermined range centered at 637 nm.

The quantum sensor system according to the present disclosure may be formed to have a shielding structure for shielding power switching noise and similar noise components. In this regard, FIG. 7 is a diagram illustrating the magnetic sensor module formed to have the shielding structure for shielding the power switching noise, according to the present specification.

With reference to FIGS. 6 and 7, a substrate of the main circuit 1200 of the quantum sensor system 2000 may be operatively coupled to the substrate 100 of the magnetic sensor module 1100. The substrate of the main circuit 1200 may be coupled to the substrate 100 of the magnetic sensor module 1100 through a connector module 100c.

In the quantum sensor system 2000, an electromagnetic wave blocking sheet 520 may be arranged on a substrate of the main circuit 1300 in such a manner to shield power noise. The electromagnetic wave blocking sheet 520 may be arranged adjacent to the main circuit 1300 to prevent a power noise signal from being introduced. The electromagnetic wave blocking sheet 520 may be arranged between the main circuit 1300 and the photodetector 420. One surface of the electromagnetic wave blocking sheet 520 may be arranged on the substrate of the main circuit 1300. The other surface of the electromagnetic wave blocking sheet 520 may be arranged on the photodetector 420.

The electromagnetic wave blocking sheet 520 may be arranged adjacent to the periphery of the photodetector 420. One surface of the electromagnetic wave blocking sheet 520 may be arranged on the substrate of the main circuit 1300. A lateral surface of the electromagnetic wave blocking sheet 520 may be arranged adjacent to the periphery of the photodetector 420.

The electromagnetic wave blocking sheet 520 may be formed as a ferrite sheet in such a manner as to shield power noise. The electromagnetic wave blocking sheet 520 may be formed as a sheet having physical properties different from those of ferrite in such a manner as to shield power noise. The electromagnetic wave blocking sheet 520 may be arranged on a lower end portion of a substrate of the main circuit board 1300 to suppress switching noise generated from a power supply portion of the main circuit 1300.

The attachment of a ferrite sheet or a sheet having different physical properties than the ferrite sheet to the lower end portion of the substrate of the main circuit 1300 makes it possible to reduce power noise and improve sensor measurement sensitivity. In this regard, a lock-in measurement standard deviation, such as a change in the resonant frequency due to a change in temperature in the quantum sensor system 2000, may be reduced from 0.108 to 0.066.

The quantum sensor system according to the present disclosure may be configured to include a resonator and an RF generator in addition to the light source and the photodetector.

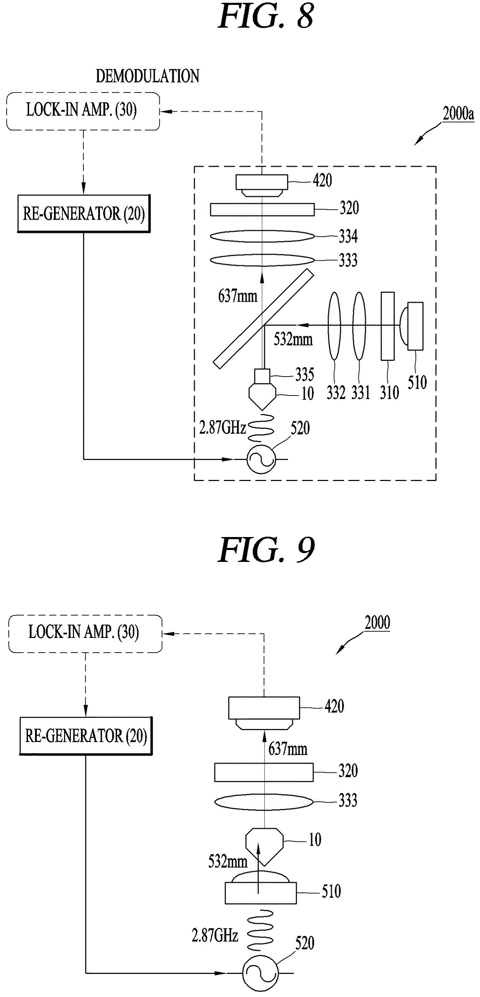

FIG. 8 is a diagram illustrating the configuration of the quantum system including a multiplicity of components, according to a first embodiment. FIG. 9 is a diagram illustrating the configuration of the quantum system including a multiplicity of components, according to a second embodiment.

With reference to FIG. 8, a quantum sensor system 2000a may be configured to include an RF generator 20, the NV diamond structure 10, the photodetector 420, the light source 510, filters 310 and 320, lenses 331, 332, 333, and 334, and a reflection surface 340. The reflection surface 340 may be configured to reflect the optical signal having the first wavelength, such as a wavelength of 532 nm, and allow the optical signal having the second wavelength, such as a wavelength of 637 nm, to pass through.

The optical signal received by the photodetector 420 may be amplified through a lock-in amplifier 30. The RF generator 20 may be configured to generate an RF signal having a specific frequency. The RF generator 20 may generate an RF signal at a specific signal level at a specific frequency on the basis of a signal level amplified through the rock-in amplifier 30. The RF signal at a specific frequency generated in the RF generator 20 may be transferred to the resonator 520. The resonator 520 may be configured to radiate an RF signal in a specific frequency band. Accordingly, the resonator 520 that radiates an RF signal in a specific frequency band may also be referred to as an antenna 520.

An RF signal at a frequency of 2.87 GHz emitted from the resonator 520 may be transferred to the NV diamond structure 10. A Gradient Index (GRIN) lens 335 may be arranged between the NV diamond structure 10 and the reflection surface 340. The GRIN lens 335 is manufactured using a polymer material crosslinking method, thereby enabling the attainment of various refractive indices.

The quantum sensor system 2000a includes the filters 310 and 320, the lenses 331, 332, 333, and 334, and the reflection surface 340, thereby rendering the structure thereof more complex and increasing the volume thereof. The quantum sensor system 2000a may be configured in such a manner that the optical signal having the first wavelength and emitted from the light source 510 is focused onto a focal point of the NV diamond structure 10. To this end, the quantum sensor system 2000a needs to include the filters 310 and 320 and the lenses 331, 332, 333, and 334 in such a manner that the optical signal having the first wavelength is focused onto the focal point of the diamond structure 10.

Each time the optical signal having the first wavelength passes through the filter 310 and the lenses 331 and 332, a phenomenon in which optical power is attenuated by approximately 20% or more occurs. Each time the optical signal having the second wavelength passes through the filter 320 and the lenses 333 and 334, the phenomenon in which optical power is attenuated by approximately 20% or more occurs. Accordingly, the optical power effectively focused onto the focal point of the NV diamond structure 10 may be at a level of 10% or less of the power emitted from the light source 510. To this end, it is necessary to increase the optical power by focusing a sufficient amount of light onto the focal point of the NV diamond structure 10 in order to secure ODMR contrast and sensitivity.

To this end, the light source 510 needs to be configured as a laser light source. However, the laser light source requires high power, and a photodiode and a lens for laser driving and output power feedback occupy a large volume. Therefore, the laser light source is subject to limitations in low-power battery operation and miniaturization. In addition, the quantum sensor system 2000a, which includes the reflection surface 340, is configured for reflective operation to keep the NV diamond structure 10 in an excited state because the use of a laser as a light source makes integration with an antenna (resonator) difficult.

With reference to FIGS. 6, 7, and 9, in the quantum sensor system 2000, the NV diamond structure 10 may be arranged over the light source 510 along the Z-axis in such a manner as to be spaced by a predetermined distance from the light source 510, thereby minimizing light attenuation. In this regard, the NV diamond structure 10 may be arranged over the light source 510, thereby directing light to be directed onto the center of the NV diamond structure 10 without light attenuation. Therefore, a sufficient amount of light from a low-power light source, such as an LED, instead of a laser light source, may be directed onto the NV diamond structure 10. Accordingly, the quantum sensor system 2000 may be driven by a low-power battery. Thus, the quantum sensor system 2000 may also be configured as a wireless communication module.

Therefore, a multiplicity of filters, for example, the filters 310 and 320, most of the lenses 331, 332, 333, and 334, and the reflection surface 340, each of which, as illustrated in FIG. 8, is used to adjust the focal point, may be removed, thereby enabling the miniaturization of the quantum sensor system 2000. In this manner, an RF antenna such as a resonator may be configured to be integrated with the substrate 100, thereby realizing the excitation state of the NV diamond structure 10 without requiring a separate reflection surface.

The quantum sensor system 2000 includes the light source 510, the NV diamond structure 10, the photodetector 420, the filter 320, and the lens 333. The RF generator 20 and the lock-in amplifier 30 may be arranged on the main circuit or the magnetic sensor module of the quantum sensor system 2000. The light source 510, the NV diamond structure 10, the photodetector 420, the filter 320, and the lens 333 may be arranged on the magnetic sensor module of the quantum sensor system 2000.

The light source 510, the filter 320, and the lens 333 are aligned along a straight line connecting the center of the NV diamond structure 10 and the center of the photodetector 420, thereby enabling the miniaturization of the quantum sensor system 2000. In addition, when an LED is used as the light source 510, the changed structure can improve a sensitivity level from 1uT or higher to 100 nT.

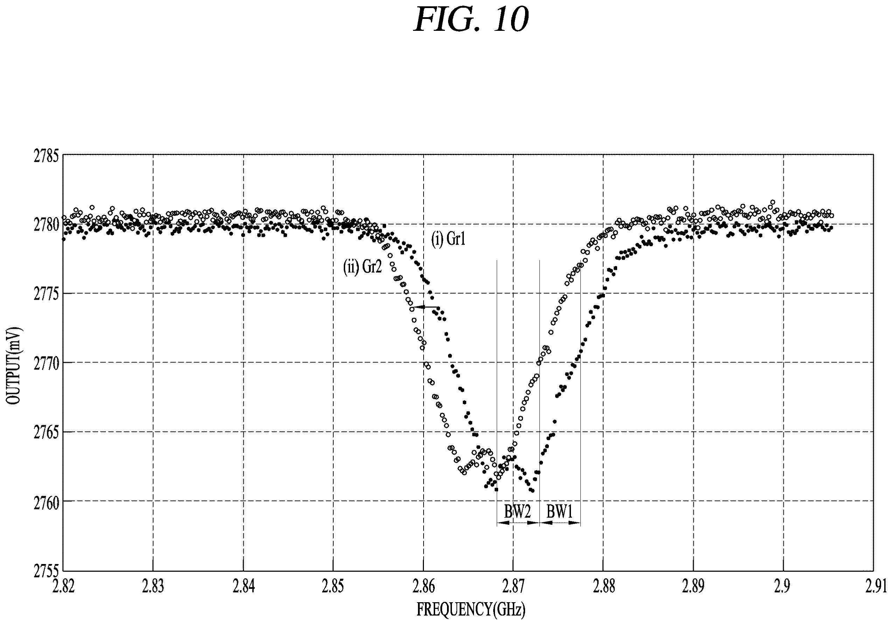

The quantum sensor system according to the present disclosure can measure the magnetic field by changing the frequency band according to the temperature value difference. In this regard, the quantum sensor system according to the present specification may be configured to compensate for the shift amount of the resonant frequency caused by the change in temperature. FIG. 10 is a graph illustrating an output voltage that varies with a change in frequency of the NV diamond structure. In FIG. 10, Curved Line 2 (Gr2) represents the output voltage that varies with the change at a higher temperature (ii) than does Curved Line 1 (Gr1) (i). From Curved Line 1 (Gr1) and Curved Line 2 (Gr2), it can be seen that a resonant frequency of the quantum sensor system transitions (is shifted) toward a lower frequency as the temperature increases.

The slope of a frequency section shifted from a specific frequency section may be measured for each of Curved Line 1 (Gr1) and Curved Line 2 (Gr2). For example, the slope of change of the output voltage that varies with the change in frequency may be measured within a specific frequency section between a frequency higher than 2.87 GHz and a frequency lower than 2.88 GHz.

In this regard, when a fine change in the magnetic field is measured, the spacing distance between band structures makes change detection difficult. Therefore, a change in a slope of an electrical property with respect to the frequency needs to be precisely measured using the lock-in amplifier. To detect the fine change in the slope of the electrical property with respect to the frequency, a change within the same frequency section needs to be detected. When the frequency of the NV diamond structure is shifted due to the change in temperature, the slope of a different frequency-shifted section is measured. Therefore, there is a possibility that a change in the magnetic field will be erroneously measured.

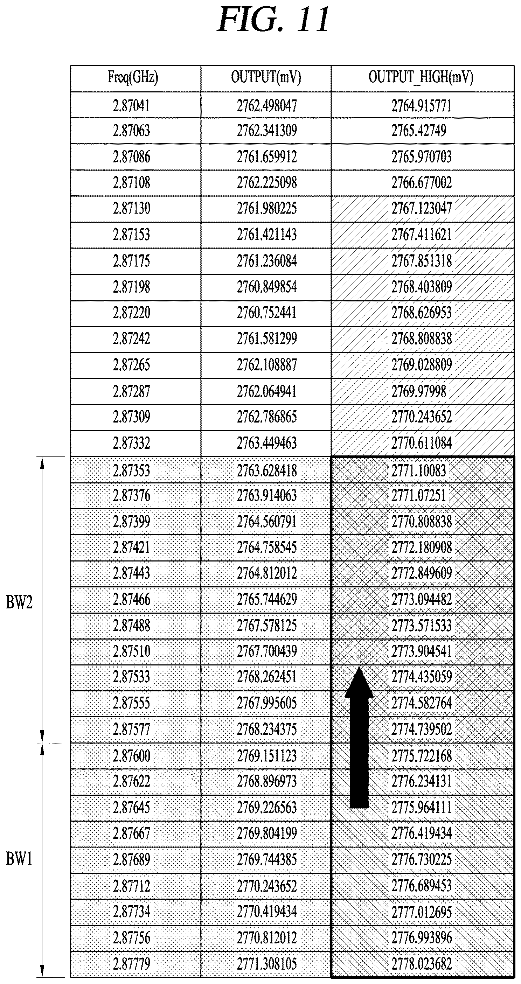

FIG. 11 is a table illustrating output voltages that result from frequency shifting under first and second temperature conditions on Curved Line 1 (Gr1) and Curved Line 2 (Gr2). With reference to FIGS. 6 to 11, the measurement of the change slope of the output voltage, which is an electrical property that varies with the frequency shifting, will be described. The outdoor air temperature measured by the first temperature sensor TS1 arranged by the main circuit 1300 may be 23.9°C, and the temperature measured by the second temperature sensor TS2 arranged adjacent to the NV diamond structure 10 of the magnetic sensor module 1100 may be 51°C. A temperature difference T is 27K, and a frequency shift value may be 2.0034 MHz, which is the product of a slope value of 0.0742 and the temperature difference T of 27K. The frequency shift spacing determined through an experiment may be set to 0.2235 MHz, and the number of shift spacings may be set to 9 ≒ 8.9637 = 2.0034 MHz / 0.2235 MHz. Therefore, the slope of a section corresponding to nine shift spacings needs to be measured for frequency-shift compensation.

The erroneously measured slope value before frequency-shift correction is 1644.689 and may be interpreted as a change in the magnetic field. The slope value after frequency-shift correction may be 1789.914. With respect to the difference between these slope values, the precision of compensation can be enhanced through additional frequency compensation rather than frequency control performed at a PCB level.

With reference to FIGS. 6, and 7 to 11, the quantum sensor system 2000 according to the present disclosure is described. The quantum sensor system 2000 may further include a lock-in amplifier 30. The lock-in amplifier 30 may be configured to amplify a signal from the photodetector 420 and output the resulting output signal. When the temperature value difference is less than a threshold temperature difference, the main circuit 1200 may measure the magnetic field that varies with a slope of the output signal that varies with the change in frequency in a first band BW1. When the temperature value difference is less than the threshold temperature difference, the slope of the output signal that varies with the change in frequency in the first frequency band BW1 exhibits linear behavior, thereby enabling precise measurement of the magnetic field.

When the temperature value difference is equal to or greater than the threshold temperature difference, the slope of the output signal that varies with the change in frequency in the first frequency band BW1 exhibits nonlinear behavior, thereby causing an error to occur in the measurement of the magnetic field. However, the slope of the output signal that varies with the change in frequency in a second frequency band BW2 narrower than the first frequency band BW1 exhibits the linear behavior, thereby enabling precise measurement of the magnetic field.

Therefore, when the temperature value difference is equal to or greater than the threshold temperature difference, the main circuit 1200 may measure the magnetic field according to the slope of the output signal that varies with the change in frequency in the second frequency band BW2. In this regard, a rise in temperature due to a light-emitting source, such as the light source 510 or the NV diamond structure 10, occurs through thermal conduction, radiation, and/or convection. In addition, the integration of a multiplicity of electronic components accompanies an additional rise in temperature, thereby necessitating compensation for the change in frequency to precisely measure the magnetic field.

When the rise in temperature due to the light source 510 occurs, the resonant frequency of the magnetic sensor module 1100 is shifted toward a narrower frequency band. Accordingly, the second frequency band BW2 for measuring the magnetic field may be set to a frequency band narrower than the first frequency band BW1.

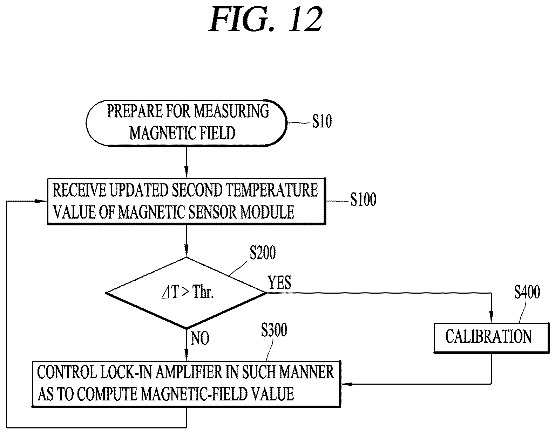

The quantum sensor system according to the present disclosure may measure the magnetic field in different ways on the basis of an updated temperature value difference between the first temperature value and the second temperature value. In this regard, FIG. 12 is a flowchart illustrating a method of controlling the quantum sensor system for measuring the magnetic field in different ways on the basis of the updated temperature value difference between the first temperature value and the second temperature value.

The method of controlling the quantum sensor system may be performed by the main circuit 1100 in FIG. 6. The method of controlling the quantum sensor system may include Step S10 of preparing for measuring the magnetic field, Step S100 of receiving the first temperature value, Step S200 of comparing the temperature value difference, Step S300 of measuring the magnetic field, and Step S400 of executing calibration.

With reference to FIGS. 6 to 12, in Step S10 of preparing for measuring the magnetic field, the main circuit 1200 acquires the first and second temperature values from the first and second temperature sensors TS1 and TS2, respectively, and prepares for measuring the magnetic field from the magnetic device. In Step S100 of receiving the first temperature value, the main circuit 1200 may receive the updated first temperature value from the magnetic sensor module 1100.

Step S200 of comparing the temperature value difference, the main circuit 1200 determines whether a temperature value difference ΔT between the first temperature value and the updated second temperature value is equal to or greater than a threshold value (Thr). When the temperature value difference ΔT is less than the threshold value (Thr), Step S300 of measuring the magnetic field is performed. In Step S300 of measuring the magnetic field, the main circuit 1200 may control the lock-in amplifier 300 in such a manner as to measure the magnetic field on the basis of a previously measured resonant frequency.

When the temperature value difference ΔT between the first temperature value and the updated second temperature value is equal to or greater than the threshold value (Thr), Step S400 of executing calibration may be performed. In Step S400 of executing calibration, the main circuit 1200 may adjust the resonant frequency of the RF generator by an amount of resonant-frequency shift in a manner that corresponds to the temperature value difference ΔT. In Step S300 of measuring the magnetic field, the main circuit 1200 may control the lock-in amplifier 300 in such a manner as to measure the magnetic field on the basis of the adjusted resonant frequency.

In this manner, the main circuit 1200 may measure the magnetic field in different ways on the basis of an updated temperature value difference between the updated first temperature value and the second temperature value. The main circuit 1200 may measure the magnetic field in a specific frequency band or change the specific frequency band for measuring the magnetic field, on the basis of the updated temperature value difference.

Specifically, when the updated temperature value difference is less than a threshold temperature difference, the main circuit 1200 may control the magnetic sensor in such a manner as to measure a magnetic field in the resonant frequency of the magnetic sensor module 1100. For example, the main circuit 1200 may measure the magnetic field according to the gradient of the output signal that varies with the change in frequency at a resonant frequency of 2.876 GHz.

When the updated temperature value difference is equal to or greater than the threshold temperature difference, the main circuit 1200 may control the magnetic sensor module 1100 in such a manner that the resonant frequency of the magnetic sensor module 1100 is shifted according to the threshold temperature difference. For example, the main circuit 1200 may measure the magnetic field according to the slope of the output signal that varies with the change in frequency at a resonant frequency of 2.876 GHz that results from shifting a previous resonant frequency of 2.873 GHz by nine shift spacings. The main circuit 1200 may control the magnetic sensor module 1100 in such a manner as to measure the magnetic field at the shifted resonant frequency.

In addition, the quantum sensor system according to the present disclosure may be configured to measure the magnetic field at the shifted resonant frequency in conjunction with the resonator and the RF generator. In this regard, the magnetic sensor module 1100 may further include the resonator 520 and the RF generator 20.

The resonator 520 may be formed on the first substrate 100 on which the light source 510 is arranged and may be configured to radiate an RF signal. The resonator 520 may be configured to radiate an RF signal toward the NV diamond structure 10 arranged on the first substrate 100. The RF generator 20 may generate an RF signal in a specific frequency band and may apply the RF signal to the resonator 520. The RF generator 20 may be configured as a voltage-controlled oscillator (VCO) that outputs a signal at a variable frequency that varies with voltage, but is not limited thereto. The RF generator 20 may be modified depending on the application. The main circuit 1200 may control the RF generator 20 in such a manner as to generate an RF signal at the shifted resonant frequency according to the change in temperature.

In this regard, the main circuit 1100 may compute a frequency shift value according to the updated temperature value difference. The main circuit 1100 may compute a value by dividing the computed frequency shift value by a minimum frequency sweep interval. The main circuit 1100 may control the RF generator 20 in such a manner as to generate an RF signal at the shifted resonant frequency corresponding to an integer multiple of frequency-step spacings that is closest to the computed value. The main circuit 1100 may control the lock-in amplifier 30 in such a manner as to measure the magnetic field at the shifted resonant frequency according to the change in temperature. The main circuit 1100 may control an operational frequency band of the lock-in amplifier 30 in such a manner as to measure the magnetic field at the shifted resonant frequency according to the change in temperature.

In this regard, the lock-in amplifier 30 may be configured to selectively amplify a signal in a specific frequency band and filter signals in adjacent frequency bands. The lock-in amplifier 30 may be configured to modulate and amplify only a signal in a variable frequency band. For example, the lock-in amplifier 30 may modulate, filter, and amplify only a signal in a specific frequency band at a frequency interval of 500 kHz or 1 MHz. Therefore, when there is a temperature increase due to the emission of light by the light source 510, the lock-in amplifier 30 may selectively amplify a signal in a frequency band including the shifted resonant frequency, in such a manner that the magnetic field is measured at a resonant frequency that is shifted by a frequency interval of 500 kHz or 1 MHz.

The quantum sensor system according to the present disclosure may change a resonant frequency and/or a wavelength of a light source, and the like on the basis of the shift amount of the resonant frequency of the magnetic sensor module 1100 or the updated temperature value difference. In this manner, when the updated temperature value difference is equal to or greater than the threshold temperature difference, the magnetic sensor module 1100 may control a change in wavelength of the light source 510 on the basis of the updated temperature value difference. When the shift amount of the resonant frequency of the magnetic sensor module 1100 is at or above the threshold value, the magnetic sensor module 1100 may control the change in the wavelength of the light source 510 on the basis of the threshold value.

When the updated temperature value difference is equal to or greater than the threshold temperature difference, the magnetic sensor module 1100 may control a change in a first resonant frequency of the resonator 520 arranged on the first substrate 100 on the basis of the updated temperature value difference. In this regard, the magnetic sensor module 1100 may control the resonant frequency of the RF generator 20 in such a manner as to change the first resonant frequency of the resonator 520.

When the shift amount of the resonant frequency of the magnetic sensor module 1100 is at or above the threshold value, the magnetic sensor module 1100 may control a change in the first resonant frequency of the resonator 520 arranged on the first substrate 100 on the basis of the threshold value.

The first temperature sensor TS1 may detect a change in outdoor air temperature due to heat generated from a processor of the main circuit 1130. The main circuit 1200 may control the magnetic sensor module 1100 in such a manner that a resonant-frequence shift value of the NV diamond structure 10 caused by the change in outdoor air temperature is decreased.

As described above, the temperature value difference, which results from a temperature increase caused by heat generated from the light source 510 or the NV diamond structure 10, is T = 27 K, and the resonant frequency may be changed to a resonant frequency of 2.873 GHz that results from shifting a resonant frequency of 2.876 GHz by nine shift spacings. When the temperature difference value between the first and second temperature values is decreased due to an increase in outdoor air temperature, an increase in the resonant frequency of the resonator 520 may be controlled. Therefore, the resonator 520 may be controlled in such a manner that a resonant frequency of 2.873 GHz is increased by predetermined shift spacings. The resonant frequency of the resonator 520 may be set to a value greater than 2.873 GHz and less than 2.876 GHz.

Therefore, even in a case where the temperature increase is not caused by heat generated from the light source 510 or the NV diamond structure 10, an influence due to the change in outdoor air temperature may be taken into account. As the resonant-frequency shift value is decreased, the sensitivity of the measurement of the magnetic field from the magnetic device 1 may be decreased. As another example, when the temperature value difference between the first and second temperature values is increased in response to a decrease in outdoor air temperature, a decrease in the resonant frequency of the resonator 520 may also be controlled.

As described, the first temperature sensor TS1 and the second temperature sensor TS2 may be arranged in different regions, respectively. The first temperature sensor TS1 may be arranged within the processor of the main circuit 1200 or within a measurement module that measures temperature. The second temperature sensor TS2 may be arranged within a processor of the second measurement module 1100 or within a second measurement module that measures temperature.

The second temperature sensor TS2 may be arranged on one side or the other side of the NV diamond structure 10. As another example, a plurality of second temperature sensors TS2 may be arranged on one side or the other side of the NV diamond structure 10.

The first temperature sensor TS1 arranged on top of the first substrate 100 may be configured using an element device having an electrical property, such as resistance, that varies with temperature. The first temperature sensor TS1 may be configured as at least one of the following: a silicon temperature sensor utilizing the dependence of a base-emitter voltage V_BE of a transistor on temperature, a thermistor, an RTD, or a thermocouple. The second temperature sensor TS2 arranged on top of the second substrate 100b may be configured using an element having an electrical property, such as resistance, that varies with temperature. The second temperature sensor TS2 may be configured as at least one of the following: a silicon temperature sensor utilizing the dependence of a base-emitter voltage V_BE of a transistor on temperature, a thermistor, an RTD, or a thermocouple.

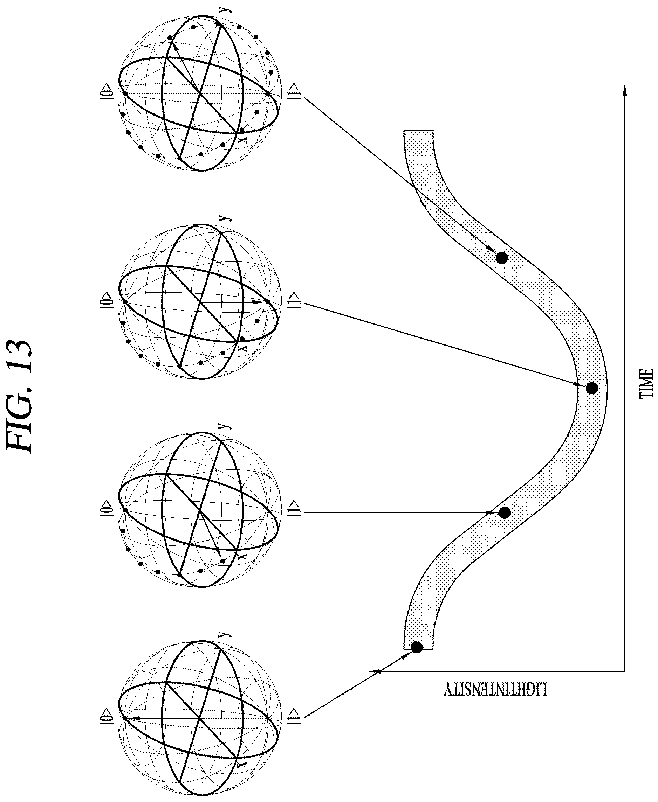

The quantum sensor system according to the present disclosure may perform spin control at a frequency corresponding to light intensity that varies with the change in temperature. In this regard, FIG. 13 is a graph illustrating the light intensity that varies with the spin state in the quantum sensor system according to the present disclosure. With reference to FIG. 13, the light intensity has a value that varies with the spin state.

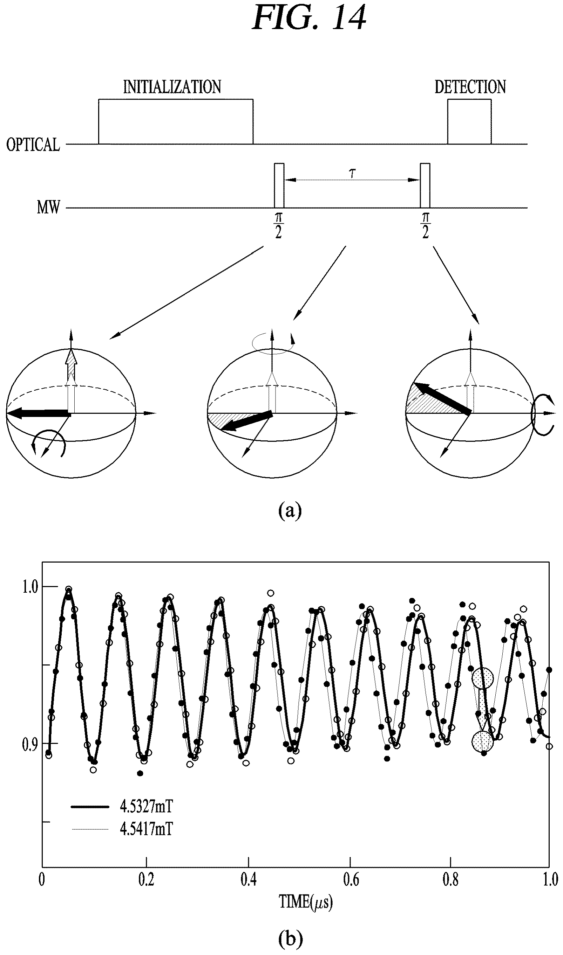

FIG. 14 is a set of conceptual diagrams, each illustrating a method of measuring continuous magnetic fields using an interferometer according to a first embodiment. With reference to (a) of FIG. 14, the spin control may be performed along horizontal axis and vertical axis directions using an RF signal between an initialization interval R1 and a detection interval R2 of the optical signal. From (b) of FIG. 14, it can be seen that respective magnetic-field values of the horizontal axis and the vertical axis periodically change over time. In addition, it can be observed that a peak value of the magnetic field along the horizontal axis and the vertical axis is gradually decreased over time.

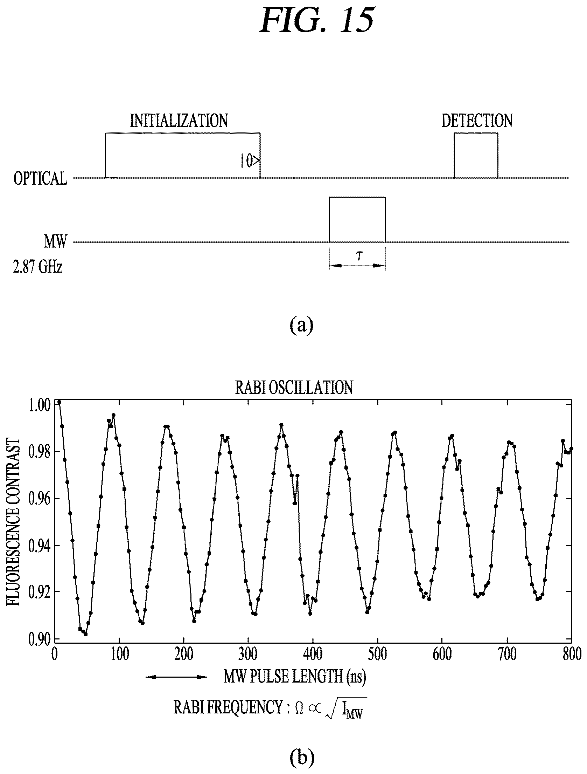

FIG. 15 is a set of conceptual diagrams, each illustrating a method of measuring an oscillation period according to a second embodiment. With reference to (a) of FIG. 15, the spin control may be performed using the RF signal between the initialization interval R1 and the detection interval R2 of the optical signal. From (b) of FIG. 15, it can be seen that fluorescence contrast is periodically changed over time. In addition, it can be seen that the peak value of the fluorescence contrast is gradually decreased over time.

With reference to FIG. 14, the Ramsey interferometry method may be used when measuring continuous magnetic fields or low-frequency magnetic fields. With reference to FIG. 15, the Rabi oscillation period may be measured for spin control using the magnetic field. When measuring the Rabi oscillation period, the spin may be controlled using an RF signal at a central frequency of the NV diamond structure for enhanced spin control. The central frequency of the NV diamond structure may be set to 2.87 GHz.

With reference to FIGS. 6, and 13 to 15, in a state where the central frequency is shifted due to the change in temperature, when the spin is controlled using the RF signal at the same frequency, the number of quanta that are changed to ±1 spin states is decreased. Therefore, the spin control is attempted at a frequency corresponding to a different light intensity than before the change in temperature. Therefore, when there is no temperature difference or the temperature difference is less than a threshold value, the spin control is executed at the existing central frequency of 2.87 GHz using a value measured by the second temperature sensor TS2 arranged adjacent to the NV diamond structure 10. When the temperature difference occurs, the spin control may be executed at a frequency of 2.87 + 74.2 (0.0742) * T GHz.

In this regard, the main circuit 1200 may track a change in an electrical property due to a single quantum spin structure associated with a single photon in the NV diamond structure 10. The main circuit 1200 may track a shift in the resonant frequency due to the single quantum spin structure associated with the single photon in the NV diamond structure 10 in conjunction with the first and second temperature sensors TS1 and TS2.

Accordingly, the main circuit 1200 may execute the spin control of the diamond structure 10 at the resonant frequency when an amount of change in temperature measured by the second temperature sensor TS2, which is arranged on the second substrate 100b in a manner that is adjacent to the NV diamond structure 10, is less than a threshold value. At this point, the resonant frequency may correspond to a central frequency of 2.87 GHz. A critical value of the amount of change in temperature may be set to a value that can be regarded as representing no change in temperature at or below a noise level. When the amount of change in temperature measured by the second temperature sensor TS2 is equal to or greater than the threshold value, the main circuit 1200 may execute the spin control of the diamond structure 10 at a frequency that results from adding an amount of change in frequency, which is in proportion to the amount of change in temperature, to the resonant frequency. The amount of change in frequency in proportion to the amount of change in temperature may correspond to 2.87 + 74.2 (0.0742) * T GHz.

With reference to FIGS. 6, and 13 to 15, the main circuit 1200 may track the shift in the resonant frequency due to the single quantum spin structure associated with the single photon in the NV diamond structure 10 in conjunction with the first and second temperature sensors TS1 and TS2. The main circuit 1200 may detect a maximum signal magnitude of the resonator during oscillation caused by the single photon according to the shift in the resonant frequency. The main circuit 1200 may minimize control errors associated with the single photon by adjusting the resonant frequency on the basis of the maximum signal magnitude and the temperature value difference.

The main circuit 1200 may track the change in an electrical property (e.g., the shift in resonant frequency) caused by a multiplicity of quantum spin structures of an ensemble NV diamond structure in conjunction with the first and second temperature sensors TS1 and TS2. The main circuit 1200 may control a multiplicity of quantum spins associated with multiple quanta in the ensemble NV diamond structure within the NV diamond structure 10. The main circuit 1200 may detect a maximum signal magnitude of the resonator during oscillation by multiple photons according to the shift in the resonant frequency. The main circuit 1200 may minimize control errors associated with multiple photons by adjusting the resonant frequency on the basis of the maximum signal magnitude and the temperature value difference. As illustrated in FIG. 6 or 7, the ensemble NV diamond structure may correspond to the NV diamond structure according to different quantum spin states.

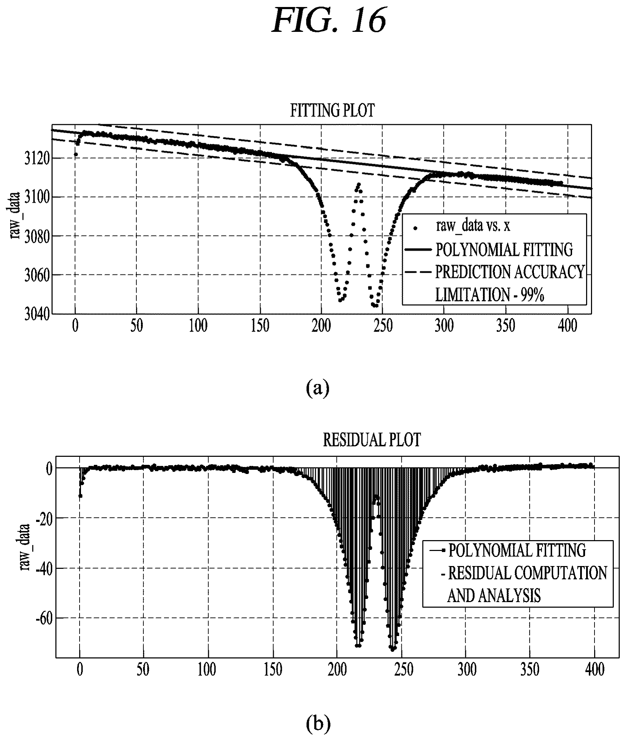

The quantum sensor system according to the present disclosure may be configured to compensate for a decrease over time in an amount of light of an optical signal from the light source. In this regard, FIG. 16 is a set of graphs, one of which illustrates the decrease over time in the amount of light of the optical signal in the quantum sensor system according to the present disclosure, and the other of which illustrates compensation for the decrease over time in the amount of light. (a) of FIG. 16 is a graph illustrating the decrease over time in the amount of light of the optical signal in the quantum sensor system. (b) of FIG. 16 is a graph illustrating the compensation for the decrease over time in the amount of light in the quantum sensor system.

The light source needs to be powered on or off to drive the quantum sensor system with lower power consumption. However, due to the characteristics of the LED light source, as illustrated in (a) of FIG. 16, a phenomenon may occur in which, even when the same current flows, the amount of light is decreased over time in a powered-on state.

With reference to FIG. 6 and (a) of FIG. 16, the main circuit 1200 may detect a rate of decrease over time in the amount of light of the optical signal from the light source 510 through a residual fitting algorithm, in conjunction with the photodetector 420. In this regard, when the X-axis is set to represent time and the Y-axis is set to represent an amount of light, the relational expression Y = -0.0685*X + 3133.1 may be established. The main circuit 1200 may detect the rate of decrease in the amount of light over time of the optical signal through the residual fitting algorithm.

The main circuit 1200 may compensate for the decrease in the amount of light over time through the residual analysis algorithm. In this regard, residual computation and analysis may be performed after deriving polynomial fitting for raw data of the optical signal from the light source 510. The residual computation and the residual analysis may be used to restore an Optically Detected Magnetic Resonance (ODMR).

With reference to FIG. 6 and (b) of FIG. 16, to compensate for the decrease over time in the amount of light of the optical signal, the main circuit 1200 may control the light source 510 in such a manner that the amount of light from the light source 510 is increased over time. Accordingly, the amount of light from the light source 510 may be maintained at a predetermined level. To this end, the main circuit 1200 may control initial driving of the light source 510 and a situation in which the amount of light is attenuated, in such a manner as to compensate for the decrease over time of the optical signal. The main circuit 1200 may control, through the residual analysis algorithm, the initial driving of the light source 510 and the situation in which the amount of light is attenuated.

The quantum sensor system for measuring the magnetic field has been described above. The technical effects of the quantum sensor system for measuring the magnetic field are described as follows.

According to the present disclosure, errors in the measurement of the magnetic field due to the change in ambient temperature can be decreased through the quantum sensor system for measuring the magnetic field through the NV diamond structure.

According to the present disclosure, the errors in the measurement of the magnetic field due to the change in ambient temperature can be decreased through the first temperature sensor provided on the main circuit and the second temperature sensor arranged adjacent to the NV diamond structure.

According to the present disclosure, the errors in the measurement of the magnetic field due to the increase in temperature in the vicinity of the NV diamond structure, which is caused by directing light having a specific wavelength, can be decreased through the first temperature sensor provided on the main circuit and the second temperature sensor arranged adjacent to the NV diamond structure.

The nature and scope of the present invention is apparent from the following detailed description. However, specific embodiments, such as preferred embodiments of the present disclosure, should be understood as merely illustrative embodiments, such that various alterations and modifications to these embodiments would be apparent, within the technical idea and scope of the present disclosure, to a person of ordinary skill in the art and should fall within the scope of the present disclosure.

## Claims

1. A quantum sensor system comprising:
a magnetic sensor module arranged to be spaced apart from a magnetic device in such a manner as to measure a magnetic field from the magnetic device and including a second temperature sensor; and
a main circuit operatively coupled to the magnetic sensor module and including a first temperature sensor,
wherein the main circuit is configured to measure the magnetic field in a specific frequency band on the basis of a temperature value difference between a first temperature value measured by the first temperature sensor and a second temperature value measured by the second temperature sensor.

2. The quantum sensor system of claim 1, wherein the magnetic sensor module comprises:
a first substrate on which a light source is arranged; and
a second substrate which is arranged to be spaced apart from the first substrate in a direction along a perpendicular axis and on which an NV diamond structure and the first temperature sensor are arranged, and
wherein a photodetector arranged to be spaced apart from the second substrate in the direction along the perpendicular axis and configured to detect a signal emitted from the NV diamond structure is provided on the magnetic sensor module or the main circuit.

3. The quantum sensor system of claim 2, further comprising:
a lock-in amplifier configured to amplify a signal from the photodetector and output the resulting output signal,
wherein, when the temperature value difference is less than a threshold temperature difference, the main circuit measures the magnetic field that varies with a slope of the output signal that varies with a change in frequency in a first band.

4. The quantum sensor system of claim 3, wherein, when the temperature value difference is equal to or greater than the threshold temperature difference, the main circuit measures the magnetic field according to the slope of the output signal that varies with the change in frequency in a second frequency band, and
wherein the second frequency band is a frequency band narrower than the first frequency band.

5. The quantum sensor system of claim 3, wherein the main circuit measures the magnetic field in the specific frequency band or changes the specific frequency band for measuring the magnetic field on the basis of an updated temperature value difference between the updated first temperature value and the second temperature value.

6. The quantum sensor system of claim 5, wherein, when the updated temperature value difference is less than the threshold temperature value, the main circuit controls the magnetic sensor module in such a manner as to measure the magnetic field at a resonant frequency of the magnetic sensor module.

7. The quantum sensor system of claim 5, wherein, when the updated temperature value difference is equal to or greater than the threshold temperature value, the main circuit controls the magnetic sensor module in such a manner that a resonant frequency of the magnetic sensor module is shifted according to the updated temperature value difference, and the main circuit controls the magnetic sensor module in such a manner as to measure the magnetic field at the shifted resonant frequency.

8. The quantum sensor system of claim 7, wherein the magnetic sensor module further comprises:
a resonator formed on the first substrate on which the light source is arranged, and configured to radiate an RF signal; and
an RF generator generating the RF signal and applying the generated RF signal to the resonator,
wherein the main circuit controls the RF generator in such a manner as to generate the RF signal at the shifted resonant frequency.

9. The quantum sensor system of claim 8, wherein the main circuit
computes a frequency shift value according to the updated temperature value difference,
computes a value by dividing the computed frequency shift value by a minimum frequency sweep interval,
controls the RF generator in such a manner as to generate the RF signal at the shifted resonant frequency corresponding to an integer multiple of frequency-step spacings that is closest to the computed value, and
controls an operational frequency band of the lock-in amplifier in such a manner as to measure the magnetic field at the shifted resonant frequency.

10. The quantum sensor system of claim 8, wherein, when the updated temperature value difference is equal to or greater than the threshold temperature value, the magnetic sensor module controls the RF generator on the basis of the updated temperature value difference in such a manner as to change a first resonant frequency of the resonator arranged on the first substrate.

11. The quantum sensor system of claim 2, wherein the first temperature sensor is arranged within a processor of the main circuit or within a measurement module that measures temperature,
wherein the second temperature sensor is arranged within the processor of the main circuit or within a second measurement module that measures temperature, and
wherein the second temperature sensor is arranged on one side or the other side of the NV diamond structure.

12. The quantum sensor system of claim 2, wherein the NV diamond structure is arranged in a slot region of the second substrate in such a manner that light emitted from the light source through the slot region of the second substrate is incident thereon.

13. The quantum sensor system of claim 2, wherein the light source is an LED light source operating in a visible light region, a laser light source, an infrared light source, or an ultraviolet light source.

14. The quantum sensor system of claim 2, wherein the first temperature sensor detects a change in outdoor air temperature due to heat generated from a processor of the main circuit,
wherein the main circuit controls the magnetic sensor module in such a manner that a resonant-frequence shift value of the NV diamond structure caused by the change in outdoor air temperature is decreased, and
wherein, as the resonant-frequence shift value is decreased, the sensitivity of the measurement of the magnetic field from the magnetic device is decreased.

15. The quantum sensor system of claim 2, wherein each of the first temperature sensor and the second temperature sensor is configured as at least one of the following: a silicon temperature sensor utilizing the dependence of a base-emitter voltage V_BE of a transistor on temperature, a thermistor, an RTD, or a thermocouple.

16. The quantum sensor system of claim 8, wherein the main circuit
tracks a shift in the resonant frequency due to a single quantum spin structure associated with a single photon in the NV diamond structure in conjunction with the first and second temperature sensors,
detects a maximum signal magnitude of the resonator during oscillation caused by the single photon according to the shift in the resonant frequency, and
minimizes control errors associated with the single photon by adjusting the resonant frequency on the basis of the maximum signal magnitude and the temperature value difference.

17. The quantum sensor system of claim 8, wherein the main circuit
tracks a shift in the resonant frequency caused by a multiplicity of quantum spin structures associated with multiple photons of an ensemble NV diamond structure within the NV diamond structure in conjunction with the first and second temperature sensors,
detects a maximum signal magnitude of the resonator during oscillation caused by the multiple photons according to the shift in the resonant frequency, and
minimizes control errors associated with the multiple photons by adjusting the resonant frequency on the basis of the maximum signal magnitude and the temperature value difference.

18. The quantum sensor system of claim 14, wherein the main circuit
executes spin control of the diamond structure at the resonant frequency when an amount of change in temperature measured by the second temperature sensor, which is arranged on the second substrate in a manner that is adjacent to the NV diamond structure, is less than a threshold value, and
executes the spin control of the diamond structure at a frequency that results from adding the amount of change in frequency, which is in proportion to the amount of change in temperature, to the resonant frequency, when the amount of change in temperature measured by the second temperature sensor is equal to or greater than the threshold value.

19. The quantum sensor system of claim 2, further comprising:
an electromagnetic wave blocking sheet arranged on a substrate of the main circuit in such a manner to shield power noise,
wherein the electromagnetic wave blocking sheet is arranged adjacent to the periphery of the photodetector, and is a ferrite sheet or a sheet having different physical properties than the ferrite sheet.

20. The quantum sensor system of claim 2, wherein the main circuit
detects a rate of decrease over time in an amount of light of an optical signal from the light source through a residual fitting algorithm, in conjunction with the photodetector, and
controls initial driving of the light source and a situation in which the amount of light is attenuated, through a residual analysis algorithm, in such a manner as to compensate for the decrease over time in the amount of light of the optical signal.
